# FASCICULE DE BREVET EUROPEEN

(11) **EP 2 267 132 B1**
(45) Date de publication et mention de la délivrance du brevet: **16.07.2014**
(21) Numéro de dépôt: 10177363.8
(22) Date de dépôt: 31.05.2000
(51) Int. Cl.: C12N 15/37, A61K 39/12, C07K 16/08, C07K 14/025

(54) **FRAGMENTS PROTEIQUES POLYEPITOPIQUES DES PROTEINES E6 ET E7 DE HPV, LEUR OBTENTION ET LEURS UTILIATIONS NOTAMMENT EN VACCINATION**
PROTEINFRAGMENTE DIE POLYEPITOPE DER PROTEINE E6 UND E7 VON HPV UMFASSEN, DEREN HERSTELLUNG UND VERWENDUNG INSBESONDERE ZUR IMPFUNG
Polyepitopic protein fragments of the HPV virus proteins E6 and E7. production and uses thereof in vaccination

(30) Priorité: 03.06.1999 FR 9907012
(43) Date de publication de la demande: 29.12.2010
(62) Demande divisionnaire de: 00938875.2
(73) Titulaire: Assistance Publique-Hopitaux, 75100 Paris RP (FR); Institut National de la Santé et de la Recherche Médicale - Inserm, 75854 Paris Cedex 13 (FR)
(72) Inventeur: Choppin, Jeannine, F-93110, Rosny sous Bois (FR); Bourgault-Villada, Isabelle, FR-75007, Paris (FR); Guille, Jean-Gérard, F-75009, Paris (FR); Connan, Francine, F-95170, Deuil-la-Barre (FR); Ferries, Estelle, F-75013, Paris (FR)
(74) Mandataire: Nederlandsch Octrooibureau

(56) Documents cités:
- EP-A- 0 257 754
- EP-A- 0 375 555
- WO-A-93/22338
- WO-A-98/23635
- WO-A1-97/41440
- WO-A2-99/27954
- DILLNER JOAKIM: "Enzyme immunoassay detection of induction of MHC class I expression by synthetic peptides from the E6 and E7 regions of human papillomavirus type 16" JOURNAL OF IMMUNOLOGICAL METHODS, ELSEVIER SCIENCE PUBLISHERS B.V.,AMSTERDAM, NL LNKD- DOI:10.1016/0022-1759(94)90088-4, vol. 167, no. 1-2, 1 janvier 1994 (1994-01-01), pages 195-205, XP002956559 ISSN: 0022-1759

### LISTE DE SEQUENCES

<110> BIOVECTOR THERAPEUTICS INSERM
<120> FRAGMENTS PROTEIQUES POLYEPITOPIQUES DE LA PROTEINE E6 OU E7 DE HPV, LEUR OBTENTION ET LEURS UTILISATIONS NOTAMMENT EN VACCINATION
<130> WOB EPIT 2
<140>
   <141>
<150> FR 9907012
   <151> 1999-06-03
<160> 18
<170> PatentIn Ver. 2.1
<210> 1
   <211> 477
   <212> ADN
   <213> Papillomavirus humain
<220>
   <221> CDS
   <222> (1)..(477)
<400> 1
   atg cac caa aag aga act gca atg ttt cag gac cca cag gag cga ccc     48
   Met His Gln Lys Arg Thr Ala Met Phe Gln Asp Pro Gln Glu Arg Pro
     1               5                  10                  15
   aga aag tta cca cag tta tgc aca gag ctg caa aca act ata cat gat     96
   Arg Lys Leu Pro Gln Leu Cys Thr Glu Leu Gln Thr Thr Ile His Asp
                20                  25                  30
   ata ata tta gaa tgt gtg tac tgc aag caa cag tta ctg cga cgt gag    144
   Ile Ile Leu Glu Cys Val Tyr Cys Lys Gln Gln Leu Leu Arg Arg Glu
            35                  40                  45
   gta tat gac ttt gct ttt cgg gat tta tgc ata gta tat aga gat ggg    192
   Val Tyr Asp Phe Ala Phe Arg Asp Leu Cys Ile Val Tyr Arg Asp Gly
        50                  55                  60
   aat cca tat gct gta tgt gat aaa tgt tta aag ttt tat tct aaa att    240
   Asn Pro Tyr Ala Val Cys Asp Lys Cys Leu Lys Phe Tyr Ser Lys Ile
    65                  70                  75                  80
   agt gag tat aga cat tat tgt tat agt ttg tat gga aca aca tta gaa    288
   Ser Glu Tyr Arg His Tyr Cys Tyr Ser Leu Tyr Gly Thr Thr Leu Glu
                    85                  90                  95
   cag caa tac aac aaa ccg ttg tgt gat ttg tta att agg tgt att aac    336
   Gln Gln Tyr Asn Lys Pro Leu Cys Asp Leu Leu Ile Arg Cys Ile Asn
               100                 105                 110
   tgt caa aag cca ctg tgt cct gaa gaa aag caa aga cat ctg gac aaa    384
   Cys Gln Lys Pro Leu Cys Pro Glu Glu Lys Gln Arg His Leu Asp Lys
           115                 120                 125
   aag caa aga ttc cat aat ata agg ggt cgg tgg acc ggt cga tgt atg    432
   Lys Gln Arg Phe His Asn Ile Arg Gly Arg Trp Thr Gly Arg Cys Met
       130                 135                 140
   tct tgt tgc aga tca tca aga aca cgt aga gaa acc cag ctg tga        477
   Ser Cys Cys Arg Ser Ser Arg Thr Arg Arg Glu Thr Gln Leu
   145 150 155
<210> 2
   <211> 158
   <212> PRT
   <213> Papillomavirus humain
<400> 2
   Met His Gln Lys Arg Thr Ala Met Phe Gln Asp Pro Gln Glu Arg Pro
   1               5                   10                  15
   Arg Lys Leu Pro Gln Leu Cys Thr Glu Leu Gln Thr Thr Ile His Asp
               20                  25                  30
   Ile Ile Leu Glu Cys Val Tyr Cys Lys Gln Gln Leu Leu Arg Arg Glu
           35                  40                  45
   Val Tyr Asp Phe Ala Phe Arg Asp Leu Cys Ile Val Tyr Arg Asp Gly
       50                  55                  60
   Asn Pro Tyr Ala Val Cys Asp Lys Cys Leu Lys Phe Tyr Ser Lys Ile
   65                  70                  75                  80
   Ser Glu Tyr Arg His Tyr Cys Tyr Ser Leu Tyr Gly Thr Thr Leu Glu
                   85                  90                  95
   Gln Gln Tyr Asn Lys Pro Leu Cys Asp Leu Leu Ile Arg Cys Ile Asn
               100                 105                 110
   Cys Gln Lys Pro Leu Cys Pro Glu Glu Lys Gln Arg His Leu Asp Lys
           115                 120                 125
   Lys Gln Arg Phe His Asn Ile Arg Gly Arg Trp Thr Gly Arg Cys Met
       130                 135                 140
   Ser Cys Cys Arg Ser Ser Arg Thr Arg Arg Glu Thr Gln Leu
   145                 150                 155
<210> 3
   <211> 90
   <212> ADN
   <213> Séquence artificielle
<220>
   <223> Description de la séquence artificielle: fragment de la séquence codant pour E6 de HPV et séquence peptidique correspondante
<220>
   <221> CDS
   <222> (1)..(90)
<400> 3
   cga ccc aga aag tta cca cag tta tgc aca gag ctg caa aca act ata      48
   Arg Pro Arg Lys Leu Pro Gln Leu Cys Thr Glu Leu Gln Thr Thr Ile
   1               5                   10                  15
   cat gat ata ata tta gaa tgt gtg tac tgc aag caa cag tta              90
   His Asp Ile Ile Leu Glu Cys Val Tyr Cys Lys Gln Gln Leu
               20                  25                  30
<210> 4
   <211> 30
   <212> PRT
   <213> séquence artificielle
<223> Description de la séquence artificielle: fragment de la séquence codant pour E6 de HPV et séquence peptidique correspondante
<400> 4
   Arg Pro Arg Lys Leu Pro Gln Leu Cys Thr Glu Leu Gln Thr Thr Ile
   1               5                   10                  15
   His Asp Ile Ile Leu Glu Cys Val Tyr Cys Lys Gln Gln Leu
               20                  25                  30
<210> 5
   <211> 66
   <212> ADN
   <213> Séquence artificielle
<220>
   <223> Description de la séquence artificielle: fragment de la séquence codant pour E6 de HPV et séquence peptidique correspondante
<220>
   <221> CDS
   <222> (1)..(66)
<400> 5
   cga cgt gag gta tat gac ttt gct ttt cgg gat tta tgc ata gta tat     48
   Arg Arg Glu Val Tyr Asp Phe Ala Phe Arg Asp Leu Cys Ile Val Tyr
   1               5                   10                  15
   aga gat ggg aat cca tat                                             66
   Arg Asp Gly Asn Pro Tyr
               20
<210> 6
   <211> 22
   <212> PRT
   <213> séquence artificielle
   <223> Description de la séquence artificielle: fragment de la séquence codant pour E6 de HPV et séquence peptidique correspondante
<400> 6
   Arg Arg Glu Val Tyr Asp Phe Ala Phe Arg Asp Leu Cys Ile Val Tyr
   1               5                   10                  15
   Arg Asp Gly Asn Pro Tyr
               20
<210> 7
   <211> 87
   <212> ADN
   <213> Séquence artificielle
<220>
   <223> Description de la séquence artificielle: fragment de la séquence codant pour E6 de HPV et séquence peptidique correspondante
<220>
   <221> CDS
   <222> (1)..(87)
<400> 7
   att agt gag tat aga cat tat tgt tat agt ttg tat gga aca aca tta    48
   Ile Ser Glu Tyr Arg His Tyr Cys Tyr Ser Leu Tyr Gly Thr Thr Leu
    1               5                  10                  15
   gaa cag caa tac aac aaa ccg ttg tgt gat ttg tta att                87
   Glu Gln Gln Tyr Asn Lys Pro Leu Cys Asp Leu Leu Ile
               20                  25
<210> 8
   <211> 29
   <212> PRT
   <213> séquence artificielle
   <223> Description de la séquence artificielle: fragment de la séquence codant pour E6 de HPV et séquence peptidique correspondante
<400> 8
   Ile Ser Glu Tyr Arg His Tyr Cys Tyr Ser Leu Tyr Gly Thr Thr Leu
    1               5                  10                  15
   Glu Gln Gln Tyr Asn Lys Pro Leu Cys Asp Leu Leu Ile
               20                  25
<210> 9
   <211> 66
   <212> ADN
   <213> séquence artificielle
<220>
   <223> Description de la séquence artificielle: fragment de la séquence codant pour E6 de HPV et séquence peptidique correspondante
<220>
   <221> CDS
   <222> (1)..(66)
<400> 9
   tgt cct gaa gaa aag caa aga cat ctg gac aaa aag caa aga ttc cat    48
   Cys Pro Glu Glu Lys Gln Arg His Leu Asp Lys Lys Gln Arg Phe His
    1               5                  10                  15
   aat ata agg ggt cgg tgg                                            66
   Asn Ile Arg Gly Arg Trp
               20
<210> 10
   <211> 22
   <212> PRT
   <213> séquence artificielle
   <223> Description de la séquence artificielle: fragment de la séquence codant pour E6 de HPV et séquence peptidique correspondante
<400> 10
   Cys Pro Glu Glu Lys Gln Arg His Leu Asp Lys Lys Gln Arg Phe His
   1               5                   10                  15
   Asn Ile Arg Gly Arg Trp
               20
<210> 11
   <211> 297
   <212> ADN
   <213> Papillomavirus humain
<220>
   <221> CDS
   <222> (1)..(297)
<400> 11
   atg cat gga gat aca cct aca ttg cat gaa tat atg tta gat ttg caa     48
   Met His Gly Asp Thr Pro Thr Leu His Glu Tyr Met Leu Asp Leu Gln
   1               5                   10                  15
   cca gag aca act gat ctc tac tgt tat gag caa tta aat gac agc tca     96
   Pro Glu Thr Thr Asp Leu Tyr Cys Tyr Glu Gln Leu Asn Asp Ser Ser
               20                  25                  30
   gag gag gag gat gaa ata gat ggt cca gct gga caa gca gaa ccg gac     144
   Glu Glu Glu Asp Glu Ile Asp Gly Pro Ala Gly Gln Ala Glu Pro Asp
           35                  40                  45
   aga gcc cat tac aat att gta acc ttt tgt tgc aag tgt gac tct acg     192
   Arg Ala His Tyr Asn Ile Val Thr Phe Cys Cys Lys Cys Asp Ser Thr
       50                  55                  60
   ctt cgg ttg tgc gta caa agc aca cac gta gac att cgt act ttg gaa     240
   Leu Arg Leu Cys Val Gln Ser Thr His Val Asp Ile Arg Thr Leu Glu
   65                  70                  75                  80
   gac ctg tta atg ggc aca cta gga att gtg tgc ccc atc tgt tct cag     288
   Asp Leu Leu Met Gly Thr Leu Gly Ile Val Cys Pro Ile Cys Ser Gln
                   85                  90                  95
   aaa cca taa                                                         297
   Lys Pro
<210> 12
   <211> 98
   <212> PRT
   <213> Papillomavirus humain
<400> 12
   Met His Gly Asp Thr Pro Thr Leu His Glu Tyr Met Leu Asp Leu Gln
   1               5                   10                  15
   Pro Glu Thr Thr Asp Leu Tyr Cys Tyr Glu Gln Leu Asn Asp Ser Ser
               20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Arg Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro
<210> 13
   <211> 69
   <212> ADN
   <213> séquence artificielle
<220>
   <223> Description de la séquence artificielle: fragment de la séquence codant pour E7 de HPV et séquence peptidique correspondante
<220>
   <221> CDS
   <222> (1)..(69)
<400> 13 .
   gga gat aca cct aca ttg cat gaa tat atg tta gat ttg caa cca gag    48
   Gly Asp Thr Pro Thr Leu His Glu Tyr Met Leu Asp Leu Gln Pro Glu
   1               5                   10                  15
   aca act gat ctc tac tgt tat                                         69
   Thr Thr Asp Leu Tyr Cys Tyr
               20
<210> 14
   <211> 23
   <212> PRT
   <213> séquence artificielle
   <223> Description de la séquence artificielle: fragment de la séquence codant pour E7 de HPV et séquence peptidique correspondante
<400> 14
   Gly Asp Thr Pro Thr Leu His Glu Tyr Met Leu Asp Leu Gln Pro Glu
   1               5                   10                  15
   Thr Thr Asp Leu Tyr Cys Tyr
               20
<210> 15
   <211> 51
   <212> ADN
   <213> séquence artificielle
<220>
   <223> Description de la séquence artificielle: fragment de la séquence codant pour E7 de HPV et séquence peptidique correspondante
<220>
   <221> CDS
   <222> (1)..(51)
<400> 15
   caa gca gaa ccg gac aga gcc cat tac aat att gta acc ttt tgt tgc     48
   Gln Ala Glu Pro Asp Arg Ala His Tyr Asn Ile Val Thr Phe Cys Cys
    1               5                  10                  15
   aag                                                                 51
   Lys
<210> 16
   <211> 17
   <212> PRT
   <213> Séquence artificielle
   <223> Description de la séquence artificielle: fragment de la séquence codant pour E7 de HPV et séquence peptidique correspondante
<400> 16
   Gln Ala Glu Pro Asp Arg Ala His Tyr Asn Ile Val Thr Phe Cys Cys
    1               5                  10                  15
   Lys
<210> 17
   <211> 57
   <212> ADN
   <213> Séquence artificielle
<220>
   <223> Description de la séquence artificielle: fragment de la séquence codant pour E7 de HPV et séquence peptidique correspondante
<220>
   <221> CDS
   <222> (1)..(57)
<400> 17
   ttg gaa gac ctg tta atg ggc aca cta gga att gtg tgc ccc atc tgt     48
   Leu Glu Asp Leu Leu Met Gly Thr Leu Gly Ile Val Cys Pro Ile Cys
    1               5                  10                  15
   tct cag aaa                                                         57
   Ser Gln Lys
<210> 18
   <211> 19
   <212> PRT
   <213> séquence artificielle
   <223> Description de la séquence artificielle: fragment de la séquence codant pour E7 de HPV et séquence peptidique correspondante
<400> 18
   Leu Glu Asp Leu Leu Met Gly Thr Leu Gly Ile Val Cys Pro Ile Cys
    1               5                  10                  15
   Ser Gln Lys

## Description

La présente invention a pour objet des fragments protéiques polyépitopiques, tels que ceux de la protéine E6 des papillomavirus humains, leur procédé d'obtention, et leurs utilisations, notamment dans le domaine de la vaccination thérapeutique ou préventive.

L'invention a plus particulièrement pour objet l'utilisation de fragments polyépitopiques d'une protéine déterminée pour la préparation de médicaments destinés à la prévention ou au traitement de pathologies dans lesquelles ladite protéine est reconnue par le système immunitaire cellulaire.

Avantageusement, lesdits fragments polyépitopiques sont tels que leur acide aminé N-terminal correspond à l'acide aminé N-terminal de l'épitope situé en amont d'un ou plusieurs autres épitopes d'une région polyépitopique de ladite protéine, et leur acide aminé C-terminal correspond à l'acide aminé C-terminal de l'épitope situé en aval du ou des épitopes susmentionnés de ladite région polyépitopique.

Ainsi, les fragments protéiques polyépitopiques susmentionnés de la présente invention correspondent avantageusement aux régions polyépitopiques d'une protéine déterminée, à savoir aux régions contenant plusieurs épitopes reconnus par les cellules T en association avec les différentes molécules du complexe majeur d'histocompatibilité (CMH), lesdites régions étant sélectionnées parmi celles ayant la caractéristique d'être dégradées *in vitro* en peptides plus courts par des protéasomes, tel que le protéasome 205, lorsque le fragment protéique testé est mis en présence dudit protéasome, notamment selon la méthode détaillée suivante. Le fragment protéique (environ 75 µg lorsqu'il s'agit d'un polypeptide d'environ 30 acides aminés) est incubé à 37°C avec environ 15µg de protéasome 20S (Calbiochem Ref 539150, La Jolla, CA, USA) dans 500 µl du tampon suivant : 20 mM Tris-HCl pH8, 0,5 mM EDTA. Des aliquots de 50 µl sont prélevés après des temps d'incubation de 24 et 48 heures, et sont analysés par chromatographie liquide haute pression (HPLC). Les produits de digestion des protéasomes sont séparés par RP-HPLC (Perkin Elmer) en utilisant une colonne C18 et un gradient acétonitrile (de 0 à 100 % contenant 0,1 % d'acide trifluoroacétique, en 90 mn, taux d'élution 0,8 ml/mn). Les produits de clivage sont détectés à 214 nm par un détecteur à absorption (759A, Applied Biosystems).

Avantageusement les régions polyépitopiques définies ci-dessus possèdent la caractéristique de contenir des acides aminés hydrophobes.

Les différents épitopes de la région polyépitopique de la protéine déterminée, et délimitant les fragments protéiques polyépitopiques, sont avantageusement sélectionnés parmi les peptides ;
- se liant à une molécule déterminée du CMH, notamment à une molécule de type HLA déterminé, et ce jusqu'à des concentrations d'environ 10⁻⁶ M à environ 10⁻¹⁰ M en peptide pour des concentrations d'environ 10⁻⁷ M en molécule HLA, notamment dans les conditions décrites ci-après,
- et formant un complexe stable avec cette molécule du CMH, à savoir notamment un complexe dans lequel ledit peptide reste lié à ladite molécule pendant au moins environ 3 heures à 37°C.

A titre d'illustration, les épitopes susmentionnés de l'invention sont sélectionnés parmi les peptides susceptibles :
- d'une part de s'associer avec les molécules du CMH, notamment par mise en oeuvre de la méthode suivante :
   - incubation (notamment pendant environ 2 heures à 25°C, puis environ 15 heures à 4°C) du peptide en présence de molécules du CMH, provenant de la lyse de cellules humaines ou animales, ou purifiées notamment par chromatographie d'affinité à partir de lignées cellulaires humaines ou animales,
   - piégeage des complexes formés lors de l'étape précédente sur un support solide recouvert d'un premier anticorps, notamment monoclonal, reconnaissant spécifiquement les molécules du CMH dans leur conformation dépendante de leur liaison audit peptide,
   - addition sur le support solide précédent d'un deuxième anticorps marqué, notamment par couplage à un marqueur radioactif, enzymatique ou fluorescent, ledit anticorps marqué reconnaissant spécifiquement soit les chaînes lourdes du CMH dans leur conformation dépendante de leur liaison au peptide, soit la chaîne légère du CMH ou la β-microglobuline se liant spécifiquement aux différentes chaînes lourdes du CMH dans leur conformation susmentionnée,
   - détection, après rinçage du support solide, de l'éventuelle présence du deuxième anticorps marqué resté fixé sur le support solide, témoignant d'un effet d'association entre les molécules du CMH et le peptide étudié,
- et, d'autre part, de former un complexe avec lesdites molécules du CMH, dont la stabilité peut être évaluée par mise en oeuvre d'une méthode de suivi dans le temps de la liaison établie entre le peptide et les molécules du CMH, cette méthode étant avantageusement effectuée selon un protocole identique à la méthode précédente, mais dans laquelle l'étape d'incubation du peptide en présence des molécules du CMH sur le support solide recouvert dudit premier anticorps, est précédée par une étape préalable d'élimination du peptide libre susceptible d'être présent dans le milieu réactionnel, notamment par lavage du support solide, ladite étape d'incubation étant effectuée (avantageusement à une température de 37°C) pendant des temps variables de 1h, 3h, 5h, 24h et 48h.

Comme mentionné ci-dessus, les épitopes de l'invention doivent être reconnus par les cellules T en association avec les molécules du CMH et s'associer à ces dernières, notamment dans le cadre de la mise en oeuvre du test de reconnaissance décrit ci-dessus. Cette association peut être faible (détectable à des concentrations en analogues peptidiques de l'ordre de 10⁻⁴ à 10⁻⁵ M), intermédiaire (détectable à des concentrations en analogues peptidiques de l'ordre de 10⁻⁶ à 10⁻⁷ M), ou forte (détectable à des concentrations en analogues peptidiques de l'ordre de 10⁻⁸ à 10⁻⁹ M). Les peptides associés aux molécules du CMH dans le cadre de la présente invention sont de préférence susceptibles de se lier pendant au moins environ 3 heures auxdites molécules du CMH.

L'invention a plus particulièrement pour objet les épitopes (encore désignés peptides ci-dessus et ci-après) tels que décrits ci-dessus et caractérisés en ce qu'ils sont sélectionnés parmi ceux susceptibles :
- d'induire *in vitro* la cytolyse par des lymphocytes T cytotoxiques, de cellules cibles présentant à leur surface le peptide susmentionné associé aux molécules du CMH, lesdits lymphocytes T cytotoxiques étant avantageusement prélevés sur un patient atteint d'une pathologie dans laquelle est impliqué le peptide étudié,
- et d'induire *in vitro* la sécrétion de cytokines (ou interleukines) par les lymphocytes T cytotoxiques susmentionnés, notamment IL-2, IL-4 ou l'interféron γ.

Le cas échéant, les épitopes susmentionnés sont choisis parmi ceux capables d'induire *in vitro* l'apparition et la croissance de lymphocytes T cytotoxiques à partir de cellules humaines ou animales, notamment à partir de cellules mononucléées issues du sang périphérique (PBMC), en présence de facteurs nécessaires à la croissance et la différenciation des cellules T cytotoxiques.

Les fragments protéiques polyépitopiques de l'invention sont davantage caractérisés en ce qu'ils sont susceptibles de contenir des épitopes CD4 reconnus par les cellules T auxiliaires en association avec les molécules du CMH de classe II, cette propriété favorisant l'induction et le maintien des cellules T CD8⁺ reconnaissant les épitopes compris dans lesdits fragments.

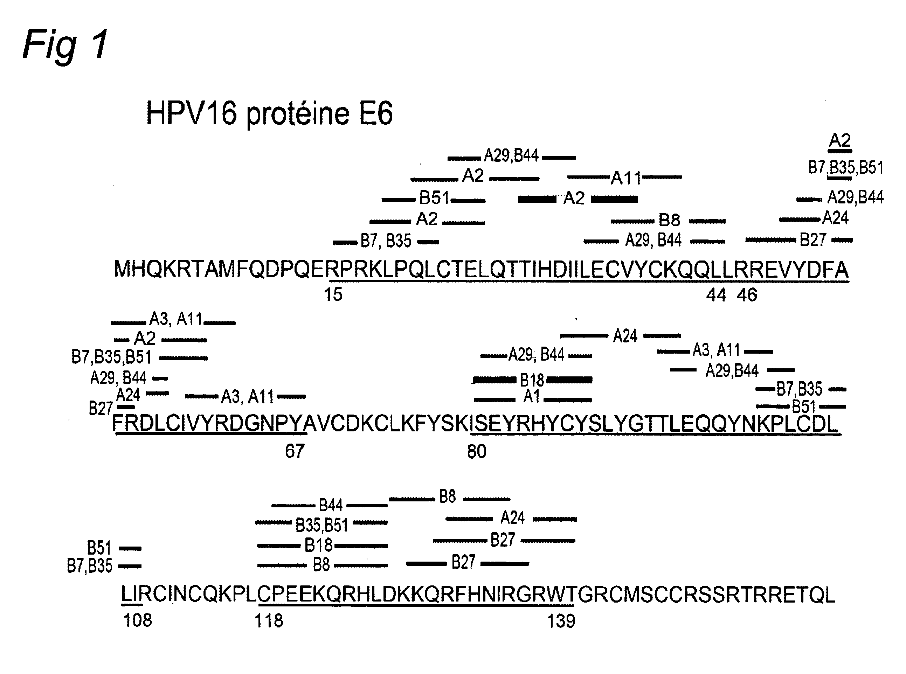

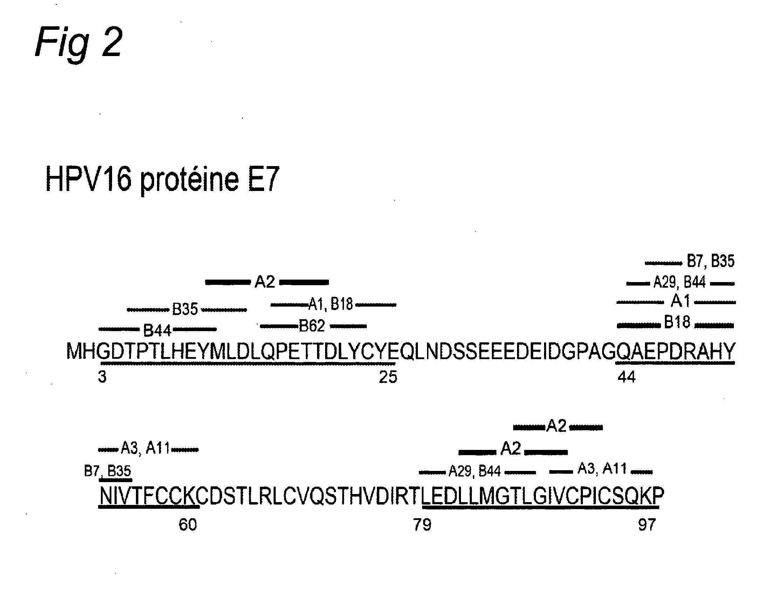

La présente invention est illustrée à l'aide des figures 1 et 2 représentant respectivement les séquences peptidiques de la protéine E6 et E7 de la souche 16 des papillomavirus humains (HPV 16), ainsi que les fragments polyépitopiques de l'invention, et les épitopes au sein de ces fragments.

L'invention a plus particulièrement pour objet les fragments polyépitopiques de la protéine E6 d'HPV, et plus particulièrement ceux de la protéine E6 représentée sur la figure 1, ou par SEQ ID NO : 2, de HPV 16, caractérisés en ce qu'ils comprennent une séquence peptidique d'environ 15 à environ 30 acides aminés, cette séquence peptidique contenant les séquences en acides aminés d'au moins 3 épitopes différents, et de préférence d'au moins 4 épitopes différents se liant de façon stable à des molécules HLA de type identique ou différent, lorsque ces épitopes sont obtenus par dégradation enzymatique de ladite séquence peptidique, notamment dans le protéasome, de sorte qu'au moins 4 molécules HLA de différents types, et de préférence au moins 5 molécules HLA de différents types, se lient à ces épitopes, ces 4 ou 5 molécules HLA étant choisies parmi celles de type A1, A2, A3, A11, A24, A29, B7, B8, B18, B27, B35, B44, B51, et B62.

Avantageusement, les fragments polyépitopiques selon l'invention sont tels que le nombre d'acides aminés de leur séquence peptidique est supérieur ou égal à 17, et inférieur ou égal à 30.

L'invention concerne plus particulièrement les fragments polyépitopiques de la protéine E6 de HPV définis ci-dessus, caractérisés en ce qu'ils comprennent une séquence peptidique d'environ 15 à 30 acides aminés, cette séquence peptidique contenant les séquences en acides aminés d'au moins 5 épitopes différents, et de préférence d'au moins 6 épitopes différents se liant de façon stable à des molécules HLA de type identique ou différent, lorsque ces épitopes sont obtenus par dégradation enzymatique de ladite séquence peptidique, notamment dans le protéasome, de sorte qu'au moins 6 molécules HLA de différents types, et de préférence au moins 7 molécules HLA de différents types se lient à ces épitopes, ces 6 ou 7 molécules HLA étant choisies parmi celles de type A1, A2, A3, A11, A24, A29, B7, B8, B18, B27, B35, B44, et B51.

Avantageusement, les fragments polyépitopiques de la protéine E6 selon l'invention, sont tels que le nombre d'acides aminés de leur séquence peptidique est supérieur ou égal à 20 (de préférence supérieur ou égal à 22), et inférieur ou égal à 30.

Avantageusement encore, les fragments polyépitopiques susmentionnés de la protéine E6 de HPV, sont caractérisés en ce qu'ils comprennent tous un épitope se liant à la molécule HLA de type B35, un épitope se liant à la molécule HLA de type B44, et un épitope se liant à la molécule HLA de type B51.

Divulgué est le fragment polyépitopique de la protéine E6 de HPV tel que défini ci-dessus, caractérisé en ce qu'il correspond au fragment de 30 acides aminés délimité par les acides aminés situés aux positions 15 et 44 de la séquence peptidique de la protéine E6 de HPV, et caractérisé par la séquence peptidique SEQ ID NO : 4 suivante :
(15)RPRKLPQLCTELQTTIHDIILECVYCKQQL(44)
ledit fragment contenant 9 épitopes se liant de façon stable à l'une au moins des 8 molécules HLA de types suivants : A2, A11, A29, B7, B8, B35, B44, ou B51, lesdits épitopes étant les suivants :
- (15)RPRKLPQL(22) se liant de façon stable aux molécules HLA de type B7, ou B35,
- (18)KLPQLCTEL(26) se liant de façon stable aux molécules HLA de type A2,
- (19)LPQLCTEL(26) se liant de façon stable aux molécules HLA de type B51,
- (21)QLCTELQTTI(30) se liant de façon stable aux molécules HLA de type A2,
- (24)TELQTTIHDI(33) se liant de façon stable aux molécules HLA de type A29, ou B44,
- (29)TIHDIILRCV(38) se liant de façon stable aux molécules HLA de type A2,
- (33)IILECVYCK(41) se liant de façon stable aux molécules HLA de type A11,
- (35)LECVYCKQQL(44) se liant de façon stable aux molécules HLA de type A29, ou B44,
- (37)CVYCKQQL(44) se liant de façon stable aux molécules HLA de type B8.

Egalement divulgué est le fragment polyépitopique de la protéine E6 de HPV tel que défini ci-dessus, caractérisé en ce qu'il correspond au fragment de 17 acides aminés délimité par les acides aminés situés aux positions 46 et 62, ou au fragment de 22 acides aminés délimité par les acides aminés situés aux positions 46 et 67 de la séquence peptidique de la protéine E6 de HPV, ce dernier fragment étant caractérisé par la séquence peptidique SEQ ID NO : 6 suivante :
(46)RREVYDFAFRDLCIVYRDGNPY(67)
ledit fragment contenant 6 épitopes se liant de façon stable à l'une au moins des 10 molécules HLA de types suivants : A2, A3, A11, A24, A29, B7, B27, B35, B44, ou B51, lesdits épitopes étant les suivants :
- (46)RREVYDFAFR(55) se liant de façon stable aux molécules HLA de type B27,
- (49)VYDFAFRDL(57) se liant de façon stable aux molécules HLA de type A24,
- (50)YDFAFRDL(57) se liant de façon stable aux molécules HLA de type A29, ou B44,
- (52)FAFRDLCIV(60) se liant de façon stable aux molécules HLA de type A2, B35, B51, ou B7,
- (54)FRDLCIVYR(62) se liant de façon stable aux molécules HLA de type A3, ou A11,
- (59)IVYRDGNPY(67) se liant de façon stable aux molécules HLA de type A3, ou A11.

Divulgué est le fragment polyépitopique de la protéine E6 de HPV tel que défini ci-dessus, caractérisé en ce qu'il correspond au fragment de 29 acides aminés délimité par les acides aminés situés aux positions 80 et 108 de la séquence peptidique de la protéine E6 de HPV, ce dernier fragment étant caractérisé par la séquence peptidique SEQ ID NO : 8 suivante :
(80)ISEYRHYCYSLYGTTLEQQYNKPLCDLLI(108)
ledit fragment contenant 6 épitopes se liant de façon stable à l'une au moins des 10 molécules HLA de types suivants : A1, A3, A11, A24, A29, B7, B18, B35, B44, ou B51, lesdits épitopes étant les suivants :
- (80)ISEYRHYCY(88) se liant de façon stable aux molécules HLA de type A1, ou B18,
- (81)SEYRHYCY(88) se liant de façon stable aux molécules HLA de type A29, ou B44,
- (87)CYSLYGTTL(95) se liant de façon stable aux molécules HLA de type A24,
- (94)TLEQQYNK(101) se liant de façon stable aux molécules HLA de type A3, ou A11,
- (95)LEQQYNKPL(103) se liant de façon stable aux molécules HLA de type A29, ou B44,
- (101)KPLCDLLI(108) se liant de façon stable aux molécules HLA de type B7, B35, ou B51.

L'invention a plus particulièrement pour objet le fragment polyépitopique de la protéine E6 de HPV tel que défini ci-dessus, caractérisé en ce qu'il correspond au fragment de 22 acides aminés délimité par les acides aminés situés aux positions 118 et 139 de la séquence peptidique de la protéine E6 de HPV, ce dernier fragment étant caractérisé par la séquence peptidique SEQ ID NO : 10 suivante :
(118)CPEEKQRHLDKKQRFHNIRGRW(139)
ledit fragment contenant 6 épitopes se liant de façon stable à l'une au moins des 7 molécules HLA de types suivants : A24, B8, B18, B27, B35, B44, ou B51, lesdits épitopes étant les suivants :
- (118)CPEEKQRHL(126) se liant de façon stable aux molécules HLA de type B8, B18, B35, B51,
- (119)PEEKQRHL(126) se liant de façon stable aux molécules HLA de type B44,
- (127)DKKQRFHNI(135) se liant de façon stable aux molécules HLA de type B8,
- (128)KKQRFHNIR(136) se liant de façon stable aux molécules HLA de type B27,
- (130)QRFHNIRGRW(139) se liant de façon stable aux molécules HLA de type B27,
- (131)RFHNIRGRW(139) se liant de façon stable aux molécules HLA de type A24.

L'invention concerne également les séquences peptidiques dérivées des fragments polyépitopiques susmentionnés de la protéine E6;
- par substitution, et/ou suppression, et/ou addition d'un ou plusieurs acides aminés, des fragments susmentionnés, et/ou
- par modification d'au moins une liaison peptidique -CO-NH- de la chaîne peptidique des fragments susmentionnés, notamment par introduction d'une liaison du type rétro, ou rétro-inverso, et/ou
- par substitution d'au moins un acide aminé de la chaîne peptidique de la séquence ou du fragment susmentionnés, par un acide aminé non protéinogénique,
lesdites séquences dérivées contenant des peptides ou pseudopeptides se liant spécifiquement à la ou aux mêmes molécules du CMH que celles se liant aux peptides contenus dans les fragments polyépitopiques susmentionnés dont elles dérivent.

Par séquence dérivée par introduction d'une liaison rétro-inverso, il faut entendre tout analogue peptidique d'un fragment susmentionné, ledit analogue étant constitué d'une chaîne peptidique dans laquelle l'un au moins des résidus d'une part est lié à au moins un résidu voisin par une liaison -NH-CO-, et d'autre part, est de chiralité opposée à celle de ce même résidu aminoacyle dans la chaîne peptidique du peptide parent (à savoir du fragment susmentionné dont elle dérive).

Par séquence dérivée par introduction d'une liaison rétro, il faut entendre tout analogue peptidique d'un fragment susmentionné, ledit analogue étant constitué d'une chaîne peptidique dans laquelle l'un au moins des résidus, est lié à au moins un résidu voisin par une liaison -NH-CO-, la chiralité de la totalité des résidus aminoacyles impliqués dans au moins une liaison -NH-CO- étant conservée par rapport aux résidus correspondant de la chaîne peptidique du peptide parent.

Il va de soi que les liaisons -CO-NH- et -NH-CO- doivent être prises en compte dans ce qui précède, dans le sens de la chaîne peptidique parente allant de l'extrémité aminoterminale (N-terminale) vers l'extrémité carboxyterminale (C-terminale).

Par "acide aminé protéinogénique", on entend, dans ce qui précède, tout acide aminé entrant dans la constitution d'une protéine ou d'un peptide naturel.

Par "acide aminé non protéinogénique", on entend par opposition à la définition précédente, tout acide aminé n'entrant pas dans la constitution d'une protéine ou d'un peptide naturel. On entend plus particulièrement par "acide aminé non protéinogénique", tout acide aminé dont le carbone portant la chaîne latérale R, à savoir le groupe -CHR-, situé entre -CO- et -NH- dans la chaîne peptidique naturelle, est remplacé par un motif n'entrant pas dans la constitution d'une protéine ou d'un peptide naturel.

L'invention a plus particulièrement pour objet les séquences dérivées telles que décrites ci-dessus, caractérisés en ce que l'une au moins des liaisons peptidiques -CO-NH- de la chaîne peptidique du peptide parent est remplacée par une liaison différente de la liaison -CO-NH-, ladite liaison différente étant notamment choisie parmi les suivantes :

| | |
|---|---|
| -CH2-NH- | (méthylène amino) ; |
| -CH₂-CH₂- | (carba) ; |
| -CO-CH₂- | (cétométhylène) ; |
| -CH₂-O- | (méthylène-oxy) ; |
| -CHOH-CH₂- | (hydroxyéthylène) ; |
| -CHOH-CHOH- | (di-hydroxyéthylène) ; |
| -CH=CH- | (E ou Z oléfine) ; |
| -CHCN-NH- | (cyanométhylène amino) ; |
| -S-CH₂- | (thiométhylène) ; |
| -CH₂-S- | (méthylène thio) ; |
| -CS-NH- | (thioamide) ; |
| -PO₂-NH | (phosphonamide) ; |
| -CHOH- | (hydroxyméthylène) ; |
| -NH-CO-NH- | (urée) ; |
| 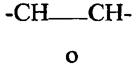 | (oxirane) ; |
| 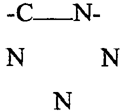 | (tétrazole) ; |
| -CH₂-CO-NH- | (ββ-homologation) ; |
| -CHOH-CH₂-NH- | (hydroxyéthylène amino) ; |
| -CO-NH-NH- | (hydrazino). |

L'invention a également pour objet les séquences nucléotidiques codant pour un fragment polyépitopique de la protéine E6, ou pour une séquence peptidique dérivée, tels que définis ci-dessus, lesdites séquences nucléotidiques étant issues de la séquence SEQ ID NO : 1 codant pour la protéine E6.

A ce titre, l'invention a plus particulièrement pour objet les séquences nucléotidiques définies ci-dessus, choisies parmi les suivantes:
- la séquence SEQ ID NO : 3, codant pour le fragment polyépitopique SEQ ID NO : 4 susmentionné de la protéine E6,
- la séquence SEQ ID NO : 7, codant pour le fragment polyépitopique SEQ ID NO : 8 susmentionné de la protéine E6,
- la séquence SEQ ID NO : 9, codant pour le fragment polyépitopique SEQ ID NO : 10 susmentionné de la protéine E6,

La divulgation a également pour objet tout vecteur, notamment plasmide, cosmide ou phage, contenant au moins une séquence nucléotidique susmentionnée placée sous le contrôle des éléments nécessaires à la transcription de ladite séquence, notamment sous le contrôle d'un promoteur et d'un terminateur de transcription.

La divulgation concerne également les cellules hôtes, notamment bactéries, virus, levures, cellules eucaryotes, transformées à l'aide d'un vecteur susmentionné selon la divulgation, de manière à intégrer de façon stable dans leur génome ou à maintenir de manière stable dans leur cytoplasme, au moins une séquence nucléotidique selon l'invention.

La divulgation concerne également tout vecteur comprenant un ou plusieurs fragments polyépitopiques et/ou une ou plusieurs séquences peptidiques dérivées tels que définis ci-dessus, ou tout vecteur comprenant une ou plusieurs séquences nucléotidiques susmentionnées, lesdits vecteurs étant choisis parmi ceux capables d'assurer une protection desdits fragments ou séquences nucléotidiques dans l'organisme et/ou leur pénétration dans les cellules de l'organisme.

Dans le cas de l'utilisation de fragments polyépitopiques et/ou de séquences peptidiques dérivées susmentionnés, de tels vecteurs sont choisis parmi les acides gras (dans le cadre de la préparation de lipopeptides), les liposomes etc.

A ce titre, l'invention a plus particulièrement pour objet tout lipopeptide caractérisé en ce qu'il comprend:
- une partie peptidique comprenant un ou plusieurs fragments protéiques polyépitopiques choisis parmi ceux définis ci-dessus, ou toute séquence peptidique dérivée desdits fragments telle que définie ci-dessus,
- et une ou plusieurs parties lipophiles, avantageusement choisies parmi celles comprenant :
   * une chaîne hydrocarbonée en C4 à C20, saturée ou insaturée, linéaire ou ramifiée,
   * ou un groupe stéroïde, le cas échéant lié à la chaîne hydrocarbonée susmentionnée,
lesdites parties lipophiles étant éventuellement associées à un court peptide vecteur (pour former ainsi des motifs lipopeptidiques vecteurs) comportant une ou plusieurs fonctions ionisées à pH physiologique, et une fonction permettant la fixation covalente de ladite chaîne hydrocarbonée et/ou dudit groupe stéroïde.

Par partie lipophile, dans ce qui précède et ce qui suit, on entend toute molécule lipophile, insoluble dans l'eau, permettant, lorsqu'elle est liée à la partie peptidique définie ci-dessus, un passage intracellulaire passif du lipopeptide obtenu, grâce aux propriétés hydrophobes de ladite molécule. Avantageusement le lipopeptide résultant de la liaison de la partie lipophile à la partie peptidique, est soluble dans l'eau.

De préférence, la chaîne hydrocarbonée des parties lipophiles, est choisie parmi celles de :
- l'acide palmitique,
- l'acide oléique,
- l'acide linoléique,
- l'acide linolénique.

De préférence également, le groupe stéroïde de la ou des parties lipophiles, est choisi parmi les dérivés du cholestérol tel que l'acide cholest-5-ényl-3-oxy acétique, ou l'acide cholest-5-ényl-3-oxycarbonique.

L'invention a plus particulièrement pour objet tout lipopeptide tel que décrit ci-dessus, caractérisé en ce que la ou les parties lipophiles sont liées de façon covalente à un ou plusieurs acides aminés de la partie peptidique.

Avantageusement, la ou les parties lipophiles sont liées de façon covalente à la fonction αNH₂ ou εNH₂ d'une lysine située en position N-terminale ou C-terminale de la partie peptidique, ou à la fonction thiol d'une cystéine, ou à toute fonction amino, alcool ou thiol éventuellement ajoutée au peptide avec un espaceur simple.

A ce titre, l'invention a plus particulièrement pour objet tout lipopeptide tel que défini ci-dessus, dans lequel la ou les parties lipophiles sont représentées par un groupe N^{α}-acétyl-Lysine N^{ε}(palmitoyl) (encore désigné par l'abréviation Ac-K(Pam)).

La présente invention a également pour objet, des micelles ou micro-agrégats d'un ou plusieurs lipopeptides différents définis ci-dessus.

Avantageusement, lesdits micelles ou micro-agrégats ont une taille inférieure à environ 1µm.

De préférence, les micelles ou micro-agrégats selon l'invention, sont tels qu'obtenus par dispersion desdits lipopeptides dans une solution d'acide acétique concentrée à environ 80%, ou tout autre solvant capable d'assurer une dispersion moléculaire des lipopeptides en solution.

Dans le cas de l'utilisation de séquences nucléotidiques définies ci-dessus selon l'invention, les vecteurs susmentionnés sont choisis parmi les virus, notamment les rétrovirus, les adénovirus et les virus associés (AAV Adeno Associated Virus).

L'invention a également pour objet les anticorps dirigés contre les fragments protéiques polyépitopiques ou les épitopes ou leurs séquences peptidiques dérivées (ou analogues) tels que définis ci-dessus, lesdits anticorps étant tels qu'obtenus par immunisation d'un animal avec au moins un des complexes susmentionnés, lesdits anticorps étant susceptibles de former un complexe avec ces fragments polyépitopiques ou ces épitopes ou leurs analogues.

Les anticorps selon l'invention sont des anticorps polyclonaux ou monoclonaux.

Les anticorps polyclonaux susmentionnés sont obtenus par immunisation d'un animal avec au moins un fragment protéique polyépitopique ou un épitope ou un analogue selon l'invention, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un fragment protéique polyépitopique ou un épitope ou un analogue selon l'invention.

Les anticorps monoclonaux selon l'invention peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec un fragment protéique polyépitopique ou un épitope ou un analogue selon l'invention, contre lesquels les lymphocytes B de l'animal sont alors capables de produire des anticorps. Ces lymphocytes producteurs d'anticorps sont ensuite fusionnés avec des cellules myélomateuses "immortelles" (notamment murines) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clones, chacun conduisant à la production d'un anticorps monoclonal dont les propriétés de reconnaissance vis-à-vis du fragment protéique polyépitopique ou épitope ou analogue de l'invention pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

L'invention concerne également l'utilisation d'un ou plusieurs anticorps susmentionnés pour la mise en oeuvre d'une méthode de diagnostic *in vitro* des pathologies susmentionnées.

A ce titre l'invention a également pour objet des trousses ou kits comprenant lesdits anticorps, pour la mise en oeuvre d'une méthode de diagnostic telle que définie ci-dessus.

L'invention concerne également les compositions pharmaceutiques, ou vaccins, caractérisés en ce qu'ils comprennent :
* a)
   - au moins un fragment polyépitopique de la protéine E6 ou E7 tel que défini ci-dessus,
   - et/ou au moins une séquence peptidique dérivée de ce fragment, telle que définie ci-dessus,
   - et/ou au moins un vecteur approprié, notamment des lipopeptides et/ou micelles définis ci-dessus, contenant au moins un fragment polyépitopique susmentionné de la protéine E6, et/ou au moins une séquence dérivée susmentionnée de ces fragments,
   en association avec un véhicule physiologiquement acceptable,
   ledit fragment protéique polyépitopique et/ou sa séquence dérivée étant le cas échéant associés à un ou plusieurs autres épitopes exogènes reconnus par des cellules T auxiliaires (encore désignés épitopes CD4 ou T helper), lesdits épitopes étant choisis notamment parmi les suivants :
   - le fragment peptidique délimité par les acides aminés situés aux positions 830 et 846 de la séquence peptidique de la toxine tétanique, ledit fragment répondant à la formule suivante : QYIKANSKFIGITELKK,
   - l'hémagglutinine (Prevost-Blondel et al., 1995, J. Virol., 62, n° 12, pp 8046-8055),
   - épitope PADRE (Alexander et al., 1994, Immunity, 1,751).
* ou b)
   - au moins une séquence nucléotidique telle que définie ci-dessus, codant pour un fragment polyépitopique susmentionné de la protéine E6,
   - et/ou au moins une séquence nucléotidique codant pour une séquence peptidique dérivée de ce fragment, telle que définie ci-dessus,
   les séquences nucléotidiques susmentionnées pouvant être utilisées nues, en tant que minigènes,
   - et/ou au moins un vecteur approprié susmentionné, choisi notamment parmi les virus tels que définis ci-dessus, contenant au moins une séquence nucléotidique susmentionnée,
   en association avec un véhicule physiologiquement acceptable,
* ou c)
   - des anticorps définis ci-dessus, dirigés contre un fragment polyépitopique de la protéine E6, et/ou contre une séquence peptidique dérivée de ces fragments, tels que définis ci-dessus, en association avec un véhicule physiologiquement acceptable.

Avantageusement les compositions pharmaceutiques, ou vaccins, susmentionnés, se présentent sous une forme administrable par voie sous-cutanée, notamment à raison de plusieurs injections (avantageusement 3 injections) d'environ 500 µg du fragment polyépitopique sous forme lipopeptidique, à environ un mois d'intervalle.

L'invention a plus particulièrement pour objet l'utilisation de fragments polyépitopiques de la protéine E6 définis ci-dessus, ou de séquences peptidiques dérivées susmentionnées, ou de séquences nucléotidiques définies ci-dessus, ou d'anticorps susmentionnés, ou de lipopeptides définis ci-dessus, pour la préparation d'un médicament ou vaccin destiné à la prévention ou au traitement de pathologies liées à l'infection d'individus par les papillomavirus humains, telles que les néoplasies cervicales intraépithéliales (CIN), le cancer invasif du col de l'utérus, les néoplasies vulvaires intraépithéliales (VIN).

L'invention concerne également les peptides ou épitopes de la protéine E6 d'HPV choisis parmi les suivants :
- (118 CPEEKQRHL(126) se liant de façon stable aux molécules HLA de type B8. B18. B35, B51.
- (119) PEEKQRHL(126) se liant de façon stable aux molécules HLA de type B44,
- (127)DKKQRFHNI(135) se liant de façon stable aux molécules HLA de type B8.
- (128)KKQRFHNIR(136) se liant de façon stable aux molécules HLA de type B27.
- (130) QRFHNIRGRW (139) se liant de façon stable aux molécules HLA de type B27.

La divulgation concerne également les peptides ou épitopes de la protéine E6 d'HPV choisis parmi les suivants:
- (19)LPQLCTEL(26) se liant de façon stable aux molécules HLA de type B51,
- (21)QLCTELQTTI(30) se liant de façon stable aux molécules HLA de type A2,
- (24)TELQTTIHDI(33) se liant de façon stable aux molécules HLA de type A29 et B44,
- (33)IILECVYCK(41) se liant de façon stable aux molécules HLA de type A11,
- (35)LECVYCKQQL(44) se liant de façon stable aux molécules HLA de type A29 et B44,
- (37)CVYCKQQL(44) se liant de façon stable aux molécules HLA de type B8,
- (46)RREVYDFAFR(55) se liant de façon stable aux molécules HLA de type B27,
- (49)VYDFAFRDL(57) se liant de façon stable aux molécules HLA de type A24,
- (50)YDFAFRDL(57) se liant de façon stable aux molécules HLA de type A29, B44,
- (52)FAFRDLCIV(60) se liant de façon stable aux molécules HLA de type A2, B35, B51,
- (54)FRDLCIVYR(62) se liant de façon stable aux molécules HLA de type A3, A11,
- (59)IVYRDGNPY(67) se liant de façon stable aux molécules HLA de type A3, A11,
- (81)SEYRHYCY(88) se liant de façon stable aux molécules HLA de type A29, B44,
- (87)CYSLYGTTL(95) se liant de façon stable aux molécules HLA de type A24,
- (94)TLEQQYNK(101) se liant de façon stable aux molécules HLA de type A3, A11,
- (95)LEQQYNKPL(103) se liant de façon stable aux molécules HLA de type A29, B44,
- (101)KPLCDLLI(108) se liant de façon stable aux molécules HLA de type B7, B35, B51,

La divulgation concerne également les séquences peptidiques dérivées des peptides susmentionnés, lesdites séquences dérivées, ou analogues, étant telles que définies ci-dessus dans le cadre des séquences dérivées des fragments protéiques polyépitopiques susmentionnés.

La divulgation a également pour objet les séquences nucléotidiques codant pour les peptides des la protéine E6 susmentionnés, à savoir :
- la séquence délimitée par les nucléotides situés aux positions 7 et 33 de la séquence SEQ ID NO : 2, codant pour (3)GDTPTLHEY(11),
- la séquence délimitée par les nucléotides situés aux positions 13 et 39 de la séquence SEQ ID NO : 2, codant pour (5)TPTLHEYML(13),
- la séquence délimitée par les nucléotides situés aux positions 43 et 69 de la séquence SEQ ID NO : 2, codant pour (15)LQPETTDLY(23),
- la séquence délimitée par les nucléotides situés aux positions 46 et 75 de la séquence SEQ ID NO : 2, codant pour (16)QPETTDLYCY(25),
- la séquence délimitée par les nucléotides situés aux positions 133 et 153 de la séquence SEQ ID NO : 2, codant pour (45)AEPDRAHY(52),
- la séquence délimitée par les nucléotides situés aux positions 136 et 165 de la séquence SEQ ID NO : 2, codant pour (46)EPDRAHYNIV(55),
- la séquence délimitée par les nucléotides situés aux positions 157 et 180 de la séquence SEQ ID NO : 2, codant pour (53)NIVTFCCK(60),
- la séquence délimitée par les nucléotides situés aux positions 235 et 261 de la séquence SEQ ID NO : 2, codant pour (79)LEDLLMGTL(87),
- la séquence délimitée par les nucléotides situés aux positions 265 et 291 de la séquence SEQ ID NO : 2, codant pour (89)IVCPICSQK(97).

La divulgation a également pour objet tout procédé de préparation de fragments polyépitopiques, d'épitopes simples (peptides susmentionnés), ou de séquences dérivées, par synthèse peptidique classique en phase liquide ou en phase solide.

En variante, les fragments polyépitopiques, épitopes simples, ou séquences peptidiques dérivées, tels que définis ci-dessus selon l'invention, peuvent être obtenus sous forme de polypeptides recombinants par transformation de cellules hôtes appropriées telles que définies ci-dessus à l'aide de vecteurs contenant une séquence nucléotidique recombinante telle que définie ci-dessus selon l'invention, et récupération, le cas échéant après purification, du polypeptide recombinant codé par ladite séquence nucléotidique et produit par les cellules hôtes susmentionnées.

## Revendications

1. Fragments polyépitopiques de la protéine E6 de HPV humaine **caractérisés en ce qu'**ils comprennent :
une séquence peptidique d'environ 15 à 30 acides aminés, préférablement avec un nombre d'acides aminés supérieur ou égal à 17, et inférieur ou égal à 30 pour les fragments polyépitopiques délimités par les acides aminés situés 118 et 139: (118)CPEEKQRHLDKKQRFHNIRGRW(139) (or SEQ ID NO :10),
- cette séquence peptidique contenant les séquences en acides aminés d'au moins 3 épitopes différents se liant de façon stable à des molécules HLA de type identique ou différent, lorsque ces épitopes sont obtenus par dégradation enzymatique de ladite séquence peptidique, notamment dans le protéasome, de sorte qu'au moins 4 molécules HLA de différents types se lient à ces épitopes, ces 4 molécules HLA étant choisies parmi celles de type A1, A2, A3, A11, A24, A29, B7, 88, B18, B27, B35, B44, B51, et B62 pour les fragments polyépitopiques de la protéine E6.

2. Fragments polyépitopiques de la protéine E6 de HPV selon la revendication 1, **caractérisés en ce qu'**ils comprennent une séquence peptidique d'environ 15 à 30 acides aminés, cette séquence peptidique contenant les séquences en acides aminés d'au moins 5 épitopes différents se liant de façon stable à des molécules HLA de type identique ou différent, lorsque ces épitopes sont obtenus par dégradation enzymatique de ladite séquence peptidique, notamment dans le protéasome, de sorte qu'au moins 6 molécules HLA de différents types se lient à ces épitopes, ces 6 molécules HLA étant choisies parmi celles de type A1, A2, A3, A11, A24, A29, B7, B8, B18, B27, B35, B44, et B51.

3. Fragments polyépitopiques de la protéine E6 de HPV selon la revendication 1 ou 2, **caractérisés en ce qu'**ils comprennent tous un épitope se liant à la molécule HLA de type B35, un épitope se liant à la molécule HLA de type B44, et un épitope se liant à la molécule HLA de type B51.

4. Fragment polyépitopique de la protéine E6 de HPV selon l'une des revendications 1 à 3, **caractérisé en ce qu'**il correspond
au fragment de 22 acides aminés délimité par les acides aminés situés aux positions 118 et 139 de la séquence peptidique de la protéine E6 de HPV, ce dernier fragment étant **caractérisé par** la séquence peptidique SEQ ID NO : 10 suivante :
(118)CPEEKQRHLDKKQRFHNIRGRW(139)
ledit fragment contenant 6 épitopes se liant de façon stable à l'une au moins des 7 molécules HLA de types suivants : A24, B8, B18, B27, B35, B44, ou B51, lesdits épitopes étant les suivants :
- (118)CPEEKQRHL(126) se liant de façon stable aux molécules HLA de type B8, B18, B35, B51,
- (119)PEEKQRHL(126) se liant de façon stable aux molécules HLA de type B44,
- (127)DKKQRFHNI(135) se liant de façon stable aux molécules HLA de type B8,
- (128)KKQRFHNIR(136) se liant de façon stable aux molécules HLA de type B27,
- (130)QRFHNIRGRW(139) se liant de façon stable aux molécules HLA de type B27,
- (131)RFHNIRGRW(139) se liant de façon stable aux molécules HLA de type A24.

5. Fragments polyépitopiques de la protéine E6 humaine selon l'une des revendications 1 à 4, **caractérisés en ce qu'**ils correspondent aux séquences peptidiques dérivées des fragments polyépitopiques définis dans l'une des revendications 1 à 4, notamment ;
- par substitution, et/ou suppression, et/ou addition d'un ou plusieurs acides aminés, des fragments susmentionnés, et/ou
- par modification d'au moins une liaison peptidique -CO-NH- de la chaîne peptidique des fragments susmentionnés, notamment par introduction d'une liaison du type rétro, ou rétro-inverso, et/ou
- par substitution d'au moins un acide aminé de la chaîne peptidique de la séquence ou du fragment susmentionnés, par un acide aminé non protéinogénique,
lesdites séquences dérivées contenant des peptides ou pseudopeptides se liant spécifiquement à la ou aux mêmes molécules du CMH que celles se liant aux peptides contenus dans les fragments polyépitopiques susmentionnés dont elles dérivent.

6. Séquences nucléotidiques codant pour un fragment polyépitopique ou pour une séquence peptidique dérivée selon l'une des revendications 1 à 5, lesdites séquences nucléotidiques étant issues de la séquence SEQ ID NO : 1 codant pour la protéine E6.

7. Séquences nucléotidiques selon la revendication 6, choisies parmi les suivantes:
- la séquence SEQ ID NO : 3, codant pour le fragment polyépitopique SEQ ID NO : 4 selon la revendication 4,
- la séquence SEQ ID NO : 7, codant pour le fragment polyépitopique SEQ ID NO : 8 selon la revendication 4,
- la séquence SEQ ID NO : 9, codant pour le fragment polyépitopique SEQ ID NO : 10 selon la revendication 4,

8. Anticorps, polyclonaux ou monoclonaux, dirigés contre un fragment polyépitopique ou contre une séquence peptidique dérivée selon l'une des revendications 1 à 5.

9. Lipopeptide **caractérisé en ce qu'**il comprend:
- une partie peptidique comprenant un ou plusieurs fragments protéiques polyépitopiques, ou une séquence peptidique dérivée desdits fragments, tels que définis dans l'une des revendications 1 à 5,
- et une ou plusieurs parties lipophiles, telles que celles comprenant :
* une chaîne hydrocarbonée en C4 à C20, saturée ou insaturée, linéaire ou ramifiée,
* ou un groupe stéroïde, le cas échéant lié à la chaîne hydrocarbonée susmentionnée, lesdites parties lipophiles étant éventuellement associées à un court peptide vecteur comportant une ou plusieurs fonctions ionisées à pH physiologique, et une fonction permettant la fixation covalente de ladite chaîne hydrocarbonée et/ou dudit groupe stéroïde.

10. Composition pharmaceutique, ou vaccin, **caractérisés en ce qu'**ils comprennent :
* a)
- au moins un fragment polyépitopique de la protéine E6 humaine défini dans l'une des revendication 1 à 4,
- et/ou au moins une séquence peptidique dérivée de ce fragment, telle que définie dans la revendication 5,
- et/ou au moins un vecteur approprié, notamment des lipopeptides selon la revendication 9 et/ou micelles, contenant au moins un fragment polyépitopique susmentionné de la protéine E6 humaine , et/ou au moins une séquence dérivée susmentionnée de ces fragments,
en association avec un véhicule physiologiquement acceptable,
ledit fragment protéique polyépitopique et/ou sa séquence dérivée étant le cas échéant associés à un ou plusieurs autres épitopes exogènes reconnus par des cellules T auxiliaires, tels que le fragment peptidique délimité par les acides aminés situés aux positions 830 et 846 de la séquence peptidique de la toxine tétanique, l'hémagglutinine,
ou l'épitope PADRE,
* ou b)
- au moins une séquence nucléotidique selon la revendication 6 ou 7, codant pour un fragment polyépitopique susmentionné de la protéine E6 humaine,
- et/ou au moins une séquence nucléotidique codant pour une séquence peptidique dérivée de ce fragment, telle que définie ci-dessus,
- et/ou au moins un vecteur approprié susmentionné, choisi notamment parmi les virus, contenant au moins une séquence nucléotidique susmentionnée,
en association avec un véhicule physiologiquement acceptable,
* ou c)
- des anticorps selon la revendication 8, dirigés contre un fragment polyépitopique de la protéine E6 humaine, et/ou contre une séquence peptidique dérivée de ces fragments, tels que définis ci-dessus.

11. Utilisation de fragments polyépitopiques de la protéine E6 humaine définis dans l'une des revendications 1 à 4, ou de séquences peptidiques dérivées selon la revendication 5, ou de séquences nucléotidiques selon la revendication 6 ou 7, ou d'anticorps selon la revendication 8, ou de lipopeptides selon la revendication 9, pour la préparation d'un médicament ou vaccin destiné à la prévention ou au traitement de pathologies liées à l'infection d'individus par les papillomavirus humains, telles que les néoplasies cervicales intraépithéliales (CIN), le cancer invasif du col de l'utérus, les néoplasies vulvaires intraépithéliales (VIN) dans le cas de la protéine E6.

12. Epitopes de la protéine E6 d'HPV humaine choisis parmi les suivants :
- (118)CPEEKQRHL(126) se liant de façon stable aux molécules HLA de type B8, B18, B35, B51,
- (119)PEEKQRHL(126) se liant de façon stable aux molécules HLA de type B44,
- (127)DKKQRFHNI(135) se liant de façon stable aux molécules HLA de type B8,
- (128)KKQRFHNIR(136) se liant de façon stable aux molécules HLA de type B27,
- (130)QRFHNIRGRW(139) se liant de façon stable aux molécules HLA de type B27.

## Patentansprüche

1. Polyepitopfragmente des Proteins E6 des humanen HPV, **dadurch gekennzeichnet, dass** sie umfassen:
eine Peptidsequenz aus ca. 15 bis 30 Aminosäuren, vorzugsweise mit einer Anzahl an Aminosäuren größer oder gleich 17 und kleiner oder gleich 30 bei den Polyepitopfragmenten die von den an 118 und 139 befindlichen Aminosäuren eingegrenzt sind: (118)CPEEKQRHLDKKQRFHNIRGRW(139) (oder SEQ ID NR: 10),
- wobei sich diese Peptidsequenz, welche die Aminosäuresequenzen von mindestens 3 verschiedenen Epitopen enthält, stabil an HLA-Moleküle eines gleichen oder unterschiedlichen Typs, wenn die Epitope durch enzymatischen Abbau der Peptidsequenz erhalten werden, insbesondere im Proteasom bindet, so dass sich mindestens 4 HLA-Moleküle unterschiedlicher Typen an diese Epitope binden, wobei die 4 HLA-Moleküle aus denjenigen des Typs A1, A2, A3, A11, A24, A29, B7, B8, B18, B27, B35, B44, B51 und B62 bei den Polyepitopfragmenten des Proteins E6 ausgewählt sind.

2. Polyepitopfragmente des Proteins E6 des HPV nach Anspruch 1, **dadurch gekennzeichnet, dass** sie eine Peptidsequenz aus ca. 15 bis 30 Aminosäuren umfassen, wobei sich diese Peptidsequenz, welche die Aminosäuresequenzen von mindestens 5 verschiedenen Epitopen enthält, stabil an HLA-Moleküle eines gleichen oder unterschiedlichen Typs, wenn die Epitope durch enzymatischen Abbau der Peptidsequenz erhalten werden, insbesondere im Proteasom bindet, so dass sich mindestens 6 HLA-Moleküle unterschiedlicher Typen an diese Epitope binden, wobei die 6 HLA-Moleküle aus denjenigen des Typs A1, A2, A3, A11, A24, A29, B7, B8, B18, B27, B35, B44 und B51 ausgewählt sind.

3. Polyepitopfragmente des Proteins E6 des HPV nach Anspruch 1 oder 2, **dadurch gekennzeichnet, dass** sie alle ein Epitop, das sich an das HLA-Molekül des Typs B35 bindet, ein Epitop, das sich an das HLA-Molekül des Typs B44 bindet, und ein Epitop umfassen, das sich an das HLA-Molekül des Typs B51 bindet.

4. Polyepitopfragment des Proteins E6 des HPV nach einem der Ansprüche 1 bis 3, **dadurch gekennzeichnet, dass** es dem Fragment von 22 Aminosäuren entspricht, das von den Aminosäuren eingegrenzt ist, die sich an den Stellen 118 und 139 der Peptidsequenz des Proteins E6 des HPV befinden, wobei dieses Fragment durch die folgende Peptidsequenz SEQ ID NR:10 gekennzeichnet ist:
(118)CPEEKQRHLDKKQRFHNIRGRW(139),
wobei das Fragment 6 Epitope enthält, die sich stabil an mindestens eines der 7 HLA-Moleküle der folgenden Typen binden: A24, B8, B18, B27, B35, B44 oder B51, wobei die Epitope die folgenden sind:
- (118)CPEEKQRHL(126), das sich stabil an die HLA-Moleküle des Typs B8, B18, B35, B51 bindet,
- (119)PEEKQRHL(126), das sich stabil an die HLA-Moleküle des Typs B44 bindet,
- (127)DKKQRFHNI(135), das sich stabil an die HLA-Moleküle des Typs B8 bindet,
- (128)KKQRFHNIR(136), das sich stabil an die HLA-Moleküle des Typs B27 bindet,
- (130)QRFHNIRGRW(139), das sich stabil an die HLA-Moleküle des Typs B27 bindet,
- (131)RFHNIRGRW(139), das sich stabil an die HLA-Moleküle des Typs A24 bindet.

5. Polyepitopfragmente des Proteins E6 nach einem der Ansprüche 1 bis 4, **dadurch gekennzeichnet, dass** sie den Peptidsequenzen entsprechen, die von den in einem der Ansprüche 1 bis 4 definierten Polyepitopfragmenten abgeleitet sind, und zwar insbesondere :
- durch Substitution und/oder Suppression und/oder Addition einer oder mehrerer Aminosäure/n der zuvor erwähnten Fragmente, und/oder
- durch Modifikation mindestens einer Peptidbindung -CO-NH- der Peptidkette der zuvor erwähnten Fragmente, insbesondere durch Einbringen einer Bindung des Typs retro oder retro-invers, und/oder
- durch Substitution mindestens einer Aminosäure der Peptidkette der vorher erwähnten Sequenz oder des vorher erwähnten Fragments durch eine nicht proteinogene Aminosäure,
wobei die abgeleiteten Sequenzen Peptide oder Pseudopeptide enthalten, die sich spezifisch an das oder dieselben Molekül/e des CMH binden wie diejenigen, die sich an die Peptide binden, die in den vorher erwähnten Polyepitopfragmenten enthalten sind, von denen sie abgeleitet sind.

6. Nukleotidsequenzen, die für ein Polyepitopfragment oder für eine nach einem der Ansprüche 1 bis 5 abgeleitete Peptidsequenz kodieren, wobei die Nukleotidsequenzen von der Sequenz SED ID NR: 1 stammen, die für das Protein E6 kodiert.

7. Nukleotidsequenzen nach Anspruch 6, die aus den folgenden ausgewählt sind:
- der Sequenz SEQ ID NR: 3, die für das Polyepitopfragment SEQ ID NR: 4 nach Anspruch 4 kodiert,
- der Sequenz SEQ ID NR: 7, die für das Polyepitopfragment SEQ ID NR: 8 nach Anspruch 4 kodiert,
- der Sequenz SEQ ID NR: 9, die für das Polyepitopfragment SEQ ID NR: 10 nach Anspruch 4 kodiert.

8. Polyklonale oder monoklonale Antikörper, die sich gegen ein Polyepitopfragment oder eine nach einem der Ansprüche 1 bis 5 abgeleitete Peptidsequenz richten.

9. Lipopeptid, **dadurch gekennzeichnet, dass** es umfasst:
- einen Peptidteil, der ein oder mehrere Polyepitopproteinfragment/e oder eine von den wie in einem der Ansprüche 1 bis 5 definierten Fragmenten abgeleitete Peptidsequenz umfasst,
- und einen oder mehrere lipophile/n Teil/e wie diejenigen, die umfassen:
* eine gesättigte oder ungesättigte, lineare oder verzweigte Kohlenwasserstoffkette in C4 bis C20,
* oder eine Steroidgruppe, die gegebenenfalls an die vorher erwähnte Kohlenwasserstoffgruppe gebunden ist, wobei die lipophilen Teile unter Umständen mit einem kurzen Trägerpeptid verbunden sind, das eine oder mehrere Funktion/en, die auf einen physiologischen pH-Wert ionisiert sind, und eine Funktion umfasst, welche die kovalente Bindung der Kohlenwasserstoffkette und/oder der Steroidgruppe zulässt.

10. Pharmazeutische Zusammensetzung oder Impfstoff, **dadurch gekennzeichnet, dass** sie umfassen:
*a)
- mindestens ein Polyepitopfragment des humanen Proteins E6, das in einem der Ansprüche 1 bis 4 definiert ist,
- und/oder mindestens eine aus diesem wie in Anspruch 5 definierten Fragment abgeleitete Peptidsequenz,
- und/oder mindestens einen geeigneten Vektor, insbesondere Lipopeptide nach Anspruch 9 und/oder Mizellen, die mindestens ein vorher erwähntes Polyepitopfragment des humanen Proteins E6 und/oder mindestens eine vorher erwähnte, von diesen Fragmenten abgeleitete Sequenz enthalten,
in Verbindung mit einem physiologisch annehmbaren Arzneistoffträger, wobei das Polyepitopproteinfragment und/oder seine abgeleitete Sequenz gegebenenfalls mit einem oder mehreren anderen exogenen Epitop/en verbunden ist, die von den T-Helferzellen erkannt werden, wie etwa das Peptidfragment, das von den Aminosäuren eingegrenzt ist, die sich an den Stellen 830 und 846 der Peptidsequenz des Tetanustoxins, des Hämagglutinins oder des Epitops PADRE befinden,
* oder b)
- mindestens eine Nukleotidsequenz nach Anspruch 6 oder 7, die für ein zuvor erwähntes Polyepitopfragment des humanen Proteins E6 kodiert,
- und/oder mindestens eine Nukleotidsequenz, die für eine von diesem Fragment wie vorstehend definiert abgeleitete Peptidsequenz kodiert,
- und/oder mindestens einen vorher erwähnten, geeigneten Vektor, der insbesondere aus Viren ausgewählt ist, der mindestens eine vorher erwähnte Nukleotidsequenz enthält,
in Verbindung mit einem physiologisch annehmbaren Arzneistoffträger,
* oder c)
- Antikörper nach Anspruch 8, die sich gegen ein Polyepitopfragment des humanen Proteins E6 und/oder gegen eine aus diesen wie vorstehend definierten Fragmenten abgeleitete Peptidsequenz richten.

11. Verwendung von in einem der Ansprüche 1 bis 4 definierten Polyepitopfragmenten des humanen Proteins E6 oder von nach Anspruch 5 abgeleiteten Peptidsequenzen oder von nach Anspruch 6 oder 7 abgeleiteten Nukleotidsequenzen oder von Antikörpern nach Anspruch 8 oder Lipopeptiden nach Anspruch 9 zur Zubereitung eines Medikaments oder Impfstoffs, das bzw. der zur Prävention oder Therapie von Krankheitsbildern bestimmt ist, die mit der Infektion von Personen mit den humanen Papillomaviren verbunden ist, wie etwa zervikalen intraepithelialen Neoplasien (CIN), invasivem Gebärmutterhalskrebs, intraepithelialen Vulvaneoplasien (VIN) im Falle des Proteins E6.

12. Epitope des Proteins E6 des humanen HPV, die aus folgenden ausgewählt sind:
(118)CPEEKQRHL(126), das sich stabil an die HLA-Moleküle des Typs B8, B18, B35, B51 bindet,
- (119)PEEKQRHL(126), das sich stabil an die HLA-Moleküle des Typs B44 bindet,
- (127)DKKQRFHNI(135), das sich stabil an die HLA-Moleküle des Typs B8 bindet,
- (128)KKQRFHNIR(136), das sich stabil an die HLA-Moleküle des Typs B27 bindet,
- (130)QRFHNIRGRW(139), das sich stabil an die HLA-Moleküle des Typs B27 bindet.

## Claims

1. Polyepitope fragments of the human HPV E6 protein **characterized in that** they comprise:
a peptide sequence of about 15 to 30 amino acids, preferably with a number of amino acids greater than or equal to 17, and less than or equal to 30 for the polyepitope fragments delimited by the amino acids located at 118 and 139:
(118)CPEEKQRHLDKKQRFHNIRGRW(139)(or SEQ ID NO: 10),
- this peptide sequence containing amino acid sequences of at least 3 different epitopes binding stably to HLA molecules of identical or different type, when these epitopes are obtained by enzymatic degradation of said peptide sequence, particularly in the proteasome, so that at least 4 HLA molecules of different types bind to these epitopes, these 4 HLA molecules being selected from among those of type A1, A2, A3, A11, A24, A29, B7, B8, B18, B27, B35, B44, B51, and B62 for the polyepitope fragments of the E6 protein.

2. Polyepitope fragments of the HPV E6 protein according to Claim 1, **characterized in that** they comprise a peptide sequence of about 15 to 30 amino acids, this peptide sequence containing amino acid sequences of at least 5 different epitopes binding stably to HLA molecules of identical or different type, when these epitopes are obtained by enzymatic degradation of said peptide sequence, particularly in the proteasome, so that at least 6 HLA molecules of different types bind to these epitopes, these 6 HLA molecules being selected from among those of type A1, A2, A3, A11, A24, A29, B7, B8, B18, B27, B35, B44, and B51.

3. Polyepitope fragments of the HPV E6 protein according to Claim 1 or 2, **characterized in that** they all comprise an epitope binding to the HLA molecule of type B35, an epitope binding to the HLA molecule of type B44, and an epitope binding to the HLA molecule of type B51.

4. Polyepitope fragment of the HPV E6 protein according to one of Claims 1 to 3, **characterized in that** it corresponds to the fragment of 22 amino acids delimited by the amino acids located in positions 118 and 139 of the peptide sequence of the HPV E6 protein, this latter fragment being **characterized by** the following peptide sequence SEQ ID NO: 10:
(118)CPEEKQRHLDKKQRFHNIRGRW(139)
said fragment containing 6 epitopes binding stably to at least one of the 7 HLA molecules of the following types: A24, B8, B18, B27, B35, B44, or B51, said epitopes being the following:
- (118)CPEEKQRHL(126) binding stably to the HLA molecules of type B8, B18, B35, B51,
- (119)PEEKQRHL(126) binding stably to the HLA molecules of type B44,
- (127)DKKQRFHNI(135) binding stably to the HLA molecules of type B8,
- (128)KKQRFHNIR(136) binding stably to the HLA molecules of type B27,
- (130)QRFHNIRGRW(139) binding stably to the HLA molecules of type B27,
- (131)RFHNIRGRW(139) binding stably to the HLA molecules of type A24.

5. Polyepitope fragments of the human E6 protein according to one of Claims 1 to 4, **characterized in that** they correspond to the peptide sequences derived from the polyepitope fragments defined in one of Claims 1 to 4, specifically:
- by substitution, and/or deletion, and/or addition of one or more amino acids, of the above-mentioned fragments, and/or
- by modification of at least one peptide bond -CO-NH- of the peptide chain of the above-mentioned fragments, particularly by introduction of a bond of the retro, or retro-inverso type, and/or
- by substitution of at least one amino acid of the peptide chain of the above-mentioned sequence or fragment, with a non-proteinogenic amino acid,
said derived sequences containing peptides or pseudopeptides binding specifically to the same MHC molecule or molecules as those binding to the peptides contained in the above-mentioned polyepitope fragments from which they derive.

6. Nucleotide sequences coding for a polyepitope fragment or for a derived peptide sequence according to one of Claims 1 to 5, said nucleotide sequences being derived from the sequence SEQ ID NO: 1 coding for the E6 protein.

7. Nucleotide sequences according to Claim 6, selected from among the following:
- the sequence SEQ ID NO: 3, coding for the polyepitope fragment SEQ ID NO: 4 according to Claim 4,
- the sequence SEQ ID NO: 7, coding for the polyepitope fragment SEQ ID NO: 8 according to Claim 4,
- the sequence SEQ ID NO: 9, coding for the polyepitope fragment SEQ ID NO: 10 according to Claim 4.

8. Polyclonal or monoclonal antibodies, directed against a polyepitope fragment or against a derived peptide sequence according to one of Claims 1 to 5.

9. Lipopeptide **characterized in that** it comprises:
- a peptide portion comprising one or more polyepitope protein fragments, or a peptide sequence derived from said fragments, as defined in one of Claims 1 to 5,
- and one or more lipophilic portions, such as those comprising:
* a C4 to C20 hydrocarbon chain, saturated or unsaturated, linear or branched,
* or a steroid group, if need be bound to the above-mentioned hydrocarbon chain, said lipophilic portions possibly being associated with a short vector peptide having one or more ionized functions at physiological pH, and a function permitting the covalent fixation of said hydrocarbon chain and/or of said steroid group.

10. Pharmaceutical composition, or vaccine, **characterized in that** it comprises:
* a)
- at least one polyepitope fragment of the human E6 protein defined in one of Claims 1 to 4,
- and/or at least one peptide sequence derived from this fragment, as defined in Claim 5,
- and/or at least one suitable vector, particularly lipopeptides according to Claim 9 and/or micelles, containing at least one above-mentioned polyepitope fragment of the human E6 protein, and/or at least one above-mentioned derived sequence of these fragments,
in association with a physiologically acceptable vehicle,
said polyepitope protein fragment and/or its derived sequence, if need be, being associated with one or more other exogenous epitopes recognized by auxiliary T cells, such as the peptide fragment delimited by the amino acids located in positions 830 and 846 of the peptide sequence of the tetanus toxin, haemagglutinin, or the PADRE epitope,
* or b)
- at least one nucleotide sequence according to Claim 6 or 7, coding for an above-mentioned polyepitope fragment of the human E6 protein,
- and/or at least one nucleotide sequence coding for a peptide sequence derived from this fragment, as defined above,
- and/or at least one above-mentioned suitable vector, particularly selected from among viruses, containing at least one above-mentioned nucleotide sequence,
in association with a physiologically acceptable vehicle,
* or c)
- antibodies according to Claim 8, directed against a polyepitope fragment of the human E6 protein, and/or against a peptide sequence derived from these fragments, as defined above.

11. Use of polyepitope fragments of the human E6 protein defined in one of Claims 1 to 4, or of derived peptide sequences according to Claim 5, or of nucleotide sequences according to Claim 6 or 7, or of antibodies according to Claim 8, or of lipopeptides according to Claim 9, for the preparation of a medicament or vaccine designed to prevent or treat pathologies linked to the infection of individuals by the human papillomaviruses, such as cervical intraepithelial neoplasias (CIN), the invasive cancer of the neck of the uterus, vulvar intraepithelial neoplasias (VIN) in the case of the E6 protein.

12. Epitopes of the human HPV E6 protein selected from among the following:
- (118)CPEEKQRHL(126) binding stably to the HLA molecules of type B8, B18, B35, B51,
- (119)PEEKQRHL(126) binding stably to the HLA molecules of type B44,
- (127)DKKQRFHNI(135) binding stably to the HLA molecules of type B8,
- (128)KKQRFHNIR(136) binding stably to the HLA molecules of type B27,
- (130)QRFHNIRGRW(139) binding stably to the HLA molecules of type B27.
